# EUROPÄISCHE PATENTSCHRIFT

(11) **EP 1 343 450 B1**
(45) Veröffentlichungstag und Bekanntmachung des Hinweises auf die Patenterteilung: **13.02.2008**
(21) Anmeldenummer: 01271194.1
(22) Anmeldetag: 11.12.2001
(51) Int. Cl.: A61K 6/00, A61K 6/033, A61K 9/00, A61K 8/19

(54) **REMINERALISIERENDE DENTAL-KLEBEFOLIE**
REMINERALISING DENTAL ADHESIVE FILM
FEUILLE ADHESIVE DENTAIRE REMINERALISANTE

(30) Priorität: 20.12.2000 DE 10063945
(43) Veröffentlichungstag der Anmeldung: 17.09.2003
(73) Patentinhaber: Henkel Kommanditgesellschaft auf Aktien, 40589 Düsseldorf (DE)
(72) Erfinder: KROPF, Christian, 40721 Hilden (DE); WÜLKNITZ, Peter, 42799 Leichlingen (DE)
(86) Internationale Anmeldenummer: PCT/EP2001/014512
(87) Internationale Veröffentlichungsnummer: WO 2002/049578

(56) Entgegenhaltungen:
- EP-A- 0 786 245
- WO-A-00/03747
- WO-A-00/18365
- WO-A-01/01930
- WO-A-01/95863
- WO-A-02/02085
- WO-A-98/26764
- DATABASE WPI Section Ch, Week 199814 Derwent Publications Ltd., London, GB; Class A96, AN 1998-148347 XP002196149 & JP 10 017449 A (SANGI KK), 20. Januar 1998 (1998-01-20)

## Beschreibung

Die Erfindung betrifft eine Klebefolie, die eine gewisse Haftung an der Zahnoberfläche oder am Zahnfleisch aufweist und in Wasser löslich oder quellbar ist und in die ein feinteiliges, in Wasser schwerlösliches Calciumsalz als remineralisierender Wirkstoff eingelagert ist.

Zur Pflege und Gesunderhaltung der Zähne werden nicht nur Reinigungsmittel wie z.B. Zahnpasten oder Mundwasser verwendet. Auch Lutschpastillen oder Kaugummipräparate, die eine längere Verweildauer im Munde haben, eignen sich dazu, bestimmte Wirkstoffe an das Zahnfleich oder an die Zahnoberfläche heranzubringen. Schließlich ist auch schon vorgeschlagen worden, Klebefolien, die auf dem Zahnfleisch oder auf der Zahnoberfläche haften, mit Wirkstoffen gegen Karies oder Parodontitis auszustatten.

Als eines der ersten Stadien der Zahnkaries werden Läsionen im Zahnschmelz und offene Dentinkanälchen (sog. Tomes Pits) beobachtet, die durch Lösungsvorgänge unter dem Einfluß von säurebildenden Bakterien entstehen. Die Öffnung der Dentinkanäle macht sich z.B. durch Zahnhalssensibilität gegenüber Temperaturschwankungen bemerkbar. Während durch Zusätze von desensibilisierenden Wirkstoffen lediglich die schmerzhaften Symptome bekämpft werden, hat man durch Zusätze, welche die Apatitlöslichkeit reduzieren, bereits versucht, die Bildung solcher Zahnoberflächen-Läsionen zu verhindern. In neuerer Zeit wurden auch bereits Vorschläge gemacht, vorhandene Schäden durch remineralisierende Zahnpflegemittel zu verringern. So wurde von Chow und Brown (in J. Dent. Res., 54, (1975), 65 - 70) vorgeschlagen, Dicalciumphosphat-dihydrat zur Remineralisation des Dentins einzusetzen. Aus US 4,097,588 war ein Mundwasser mit remineralisierender Wirkung bekannt, das mit CaHPO₄ · 2H₂O gesättigt war.

In EP 0 165 454 B1 wird Hydroxylapatit oder Fluorapatit in feinteiliger Form (unter 4 Mikrometer Teilchendurchmesser) als Komponente von Zahnpflegemitteln vorgeschlagen.

Aus EP 0 381 193 A2 sind Folien zur Applikation an der Mundschleimhaut bekannt, die einen topischen Wirkstoff, z.B. auch Natriumfluorid oder Kaliumnitrat, enthalten können.

WO 95/33441 A1 beschreibt phosphatfreie Zusammensetzungen, die feinteilige (kolloidale) Metallverbindungen, z.B. des Yttriums, Cers, Aluminiums oder Zirkoniums zur Behandlung hypersensibler Zähne enthalten und die auch in Form von Mund-Klebepflastern appliziert werden sollen.

Es bestand daher die Aufgabe, eine wirksame Applikationsform für remineralisierend wirkenden Calciumsalze, insbesondere die Fluoride, Fluorphosphate sowie Hydroxyl- und Fluorapatit zu finden, die eine lokale Remineralisation des geschädigten Zahnschmelzes bewirkt, zu finden.

Diese Aufgabe wurde erfindungsgemäß gelöst durch eine Dental-Klebefolie zur lokalen, remineralisierenden Zahnbehandlung, bestehend aus einem in Wasser löslichen oder quellbaren, am Zahn haftenden Trägermaterial und darin eingelagerten Wirkstoffen, wobei als Wirkstoffe ein feinteiliges, in Wasser schwerlösliches Calciumsalz, ausgewählt aus Fluoriden, Fluorphosphaten und Gemischen davon, das gegebenenfalls auch Hydroxyl-, Carbonat- oder Chloridionen umfassen kann, sowie eine Proteinkomponente, ausgewählt aus Gelatine, Casein, deren Hydrolysaten und Gemischen davon, enthalten ist, wobei der Wirkstoff als Kompositmaterial vorliegt in welchem die Primärpartikel der Calciumsalze an das Gerüst der Proteinkomponente assoziiert sind enthalten ist.

Die Trägerfolie kann dabei aus einem beliebigen festen, flexiblen, in Wasser löslichen oder quellbaren Material bestehen. Geeignete Materialien sind bevorzugt natürliche oder synthetische Polymere, die mit Wasser und/oder wassermischbaren Lösungsmitteln weichgemacht sind. Ein Beispiel für ein solches Material ist z.B. gemäß US 3,444,858 eine durch Wasser und Glycerin weichgemachte Gelatine. Weitere Beispiele für geeignete Trägermaterialien sind gemäß WO 00/18365 A1 z.B. Pullulan, Hydroxypropylcellulose, Hydroxyethylcellulose, Hydroxypropylmethylcellulose, Carboxymethylcellulose, Natriumalginat, Xanthan-Gum, Traganth, Guar, Akazien-Gum, Gummi Arabicum, Amylose, Hydroxypropyl-Stärke, Dextrin, Pektin, Chitin, Chitosan, Levan, Collagen, Zein, Gluten, Sojaprotein, Casein, Polyvinylpyrrolidon, Polyvinylalkohol, Polyethylenglycol, Polyacrylsäure, Methylmethacrylat/Acrylsäure-Copolymer und Gemische davon. In einer bevorzugten Ausführung der Erfindung ist als Trägerkomponente ein wasserlösliches oder wasserquellbares natürliches oder synthetisches Polymermaterial, ausgewählt aus pflanzlichen und mikrobiellen Gummen, Gelatine, Celluloseethern, Copolymeren aus Acryl- oder Methacrylsäure und Estern der Acryl- oder Methacrylsäure, Polyvinylalkohol, teilverseiftem Polyvinylacetat, Polyvinylpyrrolidon und Gemischen davon enthalten.

Bei der Zusammensetzung des Trägermaterials kommt es vor allem darauf an, daß die Wirkstoffe kontrolliert über einen längeren Zeitraum aus dem Träger freigesetzt werden, daß also das Trägermaterial im Munde unter der Einwirkung der Speichelflüssigkeit nicht zu rasch zerfällt oder sich zu rasch auflöst und der Wirkstoff verschluckt wird, bevor er am Zahn oder Zahnfleisch zur Wirkung gekommen ist.

Man kann die Desintegration oder Auflösung des Trägermaterials durch verschiedene Maßnahmen verzögern und die Freisetzung der Wirkstoffe damit gezielt kontrollieren. Solche Maßnahmen sind z.B. die Vernetzung der wasserlöslichen Polymeren, der Zusatz von weniger wasserlöslichen Polymerisaten, der Zusatz von hydrophoben Komponenten, z.B. Magnesiumstearat, oder, wie in WO 99/04764 A1 vorgeschlagen, die Verwendung von mit Gerbsäuren oder Tannin vernetzten Proteinen oder Celluloseethern.

Die Herstellung von Trägerfolien aus einem geeigneten Trägermaterial erfolgt nach bekannten Verfahren, indem man eine Lösung des Polymeren oder der Polymermischung herstellt, die Wirkstoffe darin löst oder dispergiert und diese Lösung oder Dispersion in dünner Schicht auf einer nichthaftenden, z.B. einer mit Silikon beschichteten, Unterlage trocknet. Nach der Verdunstung des Lösungsmittels läßt sich die fertige Folie von der Unterlage ablösen und gegebenenfalls in eine für die Applikation an den Zähnen geeignete Größe schneiden.

Als in Wasser schwerlösliches Calciumsalz sollen solche Salze verstanden werden, die bei 20°C zu weniger als 0,1 Gew.-% (1g/l) in Wasser löslich sind. Solche geeigneten Salze sind z.B. Calciumhydroxyphosphat (Ca₅[OH(PO₄)₃]) bzw. Hydroxylapatit, Calciumfluorphosphat (Ca₅[F(PO₄)₃]) bzw. Fluorapatit, fluordotierter Hydroxylapatit der Zusammensetzung Ca₅(PO₄)₃(OH,F) und Calciumfluorid (CaF₂) bzw. Fluorit oder Flußspat sowie andere Calciumphosphate wie Di-, Tri- oder Tetracalciumphosphat (Ca₂ P₂O₇, Ca₃(PO₄)₂, Ca₄ P₂ O₉, Oxyapatit (Ca₁₀(PO₄)₆O) oder nichtstöchiometrischer Hydroxylapatit (Ca_{5 -½(x+y)}(PO₄)₃₋ₓ(HPO₄)ₓ(OH)_{1-y}).

Als remineralisierender Wirkstoff eignet sich bevorzugt ein feinteiliges, in Wasser schwerlösliches Calciumsalz, welches ausgewählt ist aus Hydroxylapatit, Fluorapatit und Gemischen davon, da das Zahnmaterial, dessen Wiederherstellung das Ziel der Remineralisation ist, zu etwa 95 % aus Hydroxylapatit besteht.

Als besonders vorteilhaft haben sich solche wenig wasserlöslichen Calciumsalze erwiesen, die eine mittlere Teilchenfeinheit von 10-300 nm (Nanometern) ausweisen. Als Teilchenfeinheit soll hier der Durchmesser der Teilchen in Richtung ihrer größten Längenausdehnung verstanden werden. Die mittlere Teilchenfeinheit bezieht sich auf einen volumengemittelten Wert. Solche Calciumsalze lassen sich z.B. nach dem aus DE 198 58 662 A1 bekannten Verfahren in Form stäbchenförmiger Primärteilchen mit Dicken von 5-50 nm und Längen von 10-150 nm herstellen.

Im biologischen Entstehungsprozess des Zahnschmelzes sowie des Stützgewebes der Knochen lagert sich Hydroxylapatit in geordneter Weise an die Proteinmatrix im Zahn oder Knochen an, die überwiegend aus Kollagen besteht. Die Ausbildung der harten und belastungsfähigen mineralischen Struktur wird dabei durch sogenannte Matrixproteine gesteuert, die aus Kollagen und weiteren Proteinen gebildet werden, die sich an das Kollagen anlagern und so einen kontrollierten Mineralisierungsprozess, die sogenannte Biomineralisation, bewirken.

Proteine dienen auch als Schutzkolloide, die an die Oberfläche der Nanopartikel adsorbiert werden und diese an einer Koagulation und Agglomeration hindern und das Kristallwachstum verlangsamen. Auch bei der Remineralisation des geschädigten Zahnsteins kommt es darauf an, daß kein unkontrolliertes Kristallwachstum stattfindet, welches nur ein lockeres Kristallgefüge ausbilden könnte. Vielmehr sollte das Kristallwachstum gebremst und durch Proteine als Schutzkolloid kontrolliert ablaufen, damit ein dichtes und ausreichend festes Kristallgefüge gebildet werden kann.

In einer bevorzugten Ausführung enthält die erfindungsgemäße Dental-Klebefolie weiterhin eine Proteinkomponente, ausgewählt aus Proteinen, Proteinabbauprodukten und Derivaten von Proteinen oder Proteinabbauprodukten.

Als Proteine kommen dabei alle Proteine unabhängig von ihrer Herkunft infrage, also sowohl tierische wie pflanzliche Proteine. Geeignete tierische Proteine sind z.B. Kollagen, Fibroin, Elastin, Keratin, Albumin und Casein. Geeignete pflanzliche Proteine sind z.B. Weizen- und Weizenkeimproteine (Gluten), Reisprotein, Sojaprotein, Haferprotein, Erbsenprotein, Mandelprotein und Kartoffelprotein. Auch Einzellerproteine wie z.B. Hefeprotein oder Bakterienproteine sind geeignet.

Erfindungsgemäß bevorzugte Proteine sind tierische Produkte wie Kollagen, Keratin und Casein.

Gemäß einer weiteren bevorzugten Ausführungsform kann das Protein aus einer pflanzlichen oder marinen Quelle stammen.

Als Proteinabbauprodukte werden solche Produkte verstanden, die durch hydrolytischen, oxidativen oder reduktiven Abbau von wasserunlöslichen Proteinen zu Oligo- und Polypeptidstrukturen mit niedrigerem Molekulargewicht und mit einer verbesserten Wasserlöslichkeit erhältlich sind.

Der hydrolytische Abbau wasserunlöslicher Proteine ist die wichtigste Abbaumethode; sie kann unter dem katalytischen Einfluß von Säuren, Alkalien oder von Enzymen erfolgen. Bevorzugt geeignet sind vor allem solche Proteinabbauprodukte, die nicht weiter abgebaut sind als zur Erlangung der Wasserlöslichkeit erforderlich.

Zu den wenig abgebauten Proteinhydrolysaten zählt beispielsweise die im Rahmen der vorliegenden Erfindung bevorzugte Gelatine, Welche Molmassen im Bereich von 15000 bis 250000 D aufweisen kann. Gelatine ist ein Polypeptid, das vornehmlich durch Hydrolyse von Kollagen unter sauren (Gelatine Typ A) oder alkalischen (Gelatine Typ B) Bedingungen gewonnen wird. Die Gelstärke der Gelatine ist proportional zu ihrem Molekulargewicht, d. h., eine stärker hydrolysierte Gelatine ergibt eine niedriger viskose Lösung. Die Gelstärke der Gelatine wird in Bloom-Zahlen angegeben. Bei der enzymatischen Spaltung der Gelatine wird die Polymergröße stark erniedrigt, was zu sehr niedrigen Bloom-Zahlen führt.

Unter Derivaten von Proteinen und Proteinabbauprodukten werden chemisch modifizierte Proteine oder Proteinhydrolysate verstanden, die z.B. durch Acylierung freier Aminogruppen, durch Anlagerung von Ethylen- oder Propylenoxid und Hydroxyl-, Amino- oder Carboxylgruppen oder durch Alkylierung von Hydroxylgruppen des Proteins oder Proteinabbauproduktes oder eines Hydroxyalkylderivats davon, z.B. mit Epoxypropyl-trimethylammoniumchlorid oder 3-Chlor-2-hydroxypropyl-trimethylammoniumchlorid, erhältlich sind.

In einer besonders bevorzugten Ausführung ist die Proteinkomponente ausgewählt aus Gelatine, Casein, deren Hydrolysaten und Gemischen davon. Die erfindungsgemäße Dental-Klebefolie kann z.B. als Trägermaterial überwiegend aus einer Proteinkomponente, z.B. aus Gelatine oder Kollagen, bestehen. Wenn aber als Trägermaterial ein anders Material, z.B. ein Pflanzen-Gum, ein Einzeller-Biopolymer (Xanthan-Gum, Pullulan), ein Cellulose- oder Stärkeether, ein Polyvinylpyrrolidon oder ein Gemisch aus Celluloseether, Polyvinylacetat und Polyacrylsäure verwendet wird, dann sollte bevorzugt eine Proteinkomponente darin in einer Menge von wenigstens 1 Gew.-%, bevorzugt von 1-20 Gew.-% enthalten sein.

Erfindungsgemäß ist der Wirkstoff als Kompositmaterial aus dem in Wasser schwerlöslichen Calciumsalz und einer Proteinkomponente, ausgewählt aus Gelatine casein deren Hydrolysolen und Gemischen davon enthalten. Als Kompositmaterialien werden dabei Verbundstoffe verstanden, welche die schwerlöslichen Calciumsalze und die Proteinkomponenten umfassen und mikroskopisch heterogene, makroskopisch aber homogen erscheinende Aggregate darstellen, in welchen die Primärpartikel der Calciumsalze an das Gerüst der Proteinkomponente assoziiert vorliegen. Der Anteil der Proteinkomponenten in solchen Kompositmaterialien liegt zwischen 0,1 und 60 Gew.% bevorzugt jedoch zwischen 1,0 und 20 Gew.-%, bezogen auf das Gewicht des Kompositmaterials.

Die Herstellung von Kompositmaterialien aus Hydroxylapatit und Kollagen wird z.B. von R.Z. Wang et al., J. Mater. Sci. Lett. 14 (1995), 490 beschrieben. Die dort vorliegenden Hydroxylapatit-Teilchen haben eine Teilchenfeinheit von 2-10 nm und gehören daher dem Bereich der amorphen oder teilweise röntgenamorphen Stoffe an. Besser geeignet sind Hydroxylapatit-Nanopartikel, die eine deutlich erkennbare kristalline Morphologie aufweisen, deren Teilchenfeinheit daher im Bereich von 10-300 nm liegt. Ebenfalls besser geeignet sind Kompositmaterialien, bei denen die feinteiligen schwerlöslichen Calciumsalze mit Teilchenfeinheiten von 10-300 nm zusammen mit feinteiligen Proteinen, Proteinhydrolysaten oder Derivaten davon eine räumliche Struktur derart bilden, daß die feinteiligen Calciumsalze der Proteinstruktur aufgelagert sind, diese quasi räumlich abbilden. Aus solchen bevorzugt geeigneten nanopartikulären Calciumsalzen und Proteinkomponenten bestehende Kompositmaterialien führen zu einer besonders effektiven Biomineralisation.

Erfindungsgemäß geeignete Kompositmaterialien können durch Fällung aus wäßrigen Lösungen wasserlöslicher Calciumsalze mit wäßrigen Lösungen wasserlöslicher Phosphat und/oder Fluoridsalze in Gegenwart von Proteinkomponenten hergestellt werden.

Dies erfolgt vorzugsweise in der Weise, daß die Proteinkomponenten in reiner, gelöster oder kolloidaler Form der alkalischen wäßrigen Phosphat- und / oder Fluorid-Salzlösung oder der alkalischen Lösung des Calciumsalzes vor der Fällungsreaktion beigefügt werden. Alternativ können die Proteinkomponenten in reiner, gelöster oder kolloidaler Form vorgelegt und anschließend nacheinander in beliebiger Reihenfolge oder gleichzeitig mit der alkalischen Calcium-Salzlösung sowie der alkalischen Phosphat- und / oder Fluorid-Salzlösung versetzt werden.

Bei den Herstellverfahren kann die Zusammenfügung der einzelnen Komponenten grundsätzlich in allen möglichen Reihenfolgen erfolgen.' Als Alkalisierungsmittel wird bevorzugt Ammoniak verwendet. Bei allen Fällungsreaktionen dieser Art soll der pH-Wert dem gefällten System oberhalb von pH=5 liegen.

Eine weitere Variante des Herstellverfahrens besteht darin, daß man die Fällung aus einer sauren Lösung eines wasserlöslichen Calciumsalzes zusammen mit einer stöchiometrischen Menge eines wasserlöslichen Phosphat- und / oder Fluoridsalzes oder aus einer sauren Lösung von Hydroxylapatit mit einem pH-Wert unterhalb von 5, bevorzugt bei einem pH-Wert unterhalb von 3, durch Anheben des pH-Werts mit wäßrigem Alkali oder Ammoniak auf einen Wert oberhalb von 5 in Gegenwart der Proteinkomponenten durchführt.

Eine weitere Verfahrensvariante besteht darin, daß man nanopartikuläre Calciumsalze in reiner oder dispergierter Form oder durch Fällungsreaktionen aus wässrigen Lösungen wasserlöslicher Calciumsalze und wässrigen Lösungen wasserlösliclier Phosphat- und / oder Fluoridsalze hergestellte Dispersionen nanopartikulärer Calciumsalze mit den Proteinkomponenten, letztere bevorzugt in gelöster oder dispergierter Form, versetzt, wobei bei der Zugabe eine beliebige Reihenfolge gewählt werden kann.
Bevorzugt wird die Lösung oder Dispersion der Proteinkomponente vorgelegt und eine Dispersion des nanopartikulären Calciumsalzes zugefügt.

Bei allen Verfahren, im Verlauf derer eine Fällung von Apatit stattfindet, empfiehlt es sich, den pH-Wert oberhalb von 5, zu halten.

Bei allen genannten Herstellverfahren kann die entstehende Dispersion des Kompositmaterials nach Bedarf durch dem Fachmann bekannte Verfahren, wie z. B. Filtration oder Zentrifugation, vom Lösungsmittel und den übrigen Bestandteilen des Reaktionsgemischs abgetrennt und durch anschließende Trocknung, z. B. durch Gefriertrocknung, in lösungsmittelfreier Form isoliert werden.

Als Lösungsmittel wird bei allen Herstellungsprozessen bevorzugt Wasser verwendet, jedoch können in einzelnen Schritten der Herstellung auch organische Lösungsmittel wie z. B. Alkohole mit 1 bis 4 C-Atomen oder Glycerin verwendet werden.

In einer besonderen Ausführungsform der Erfindung können die feinteiligen Calciumsalz-Primärteilchen oder die in den Kompositmaterialien vorliegenden feinteiligen Calciumsalz-Primärteilchen von einem oder mehreren Oberflächenmodifikationsmitteln umhüllt sein.

Dadurch kann beispielsweise die Herstellung von Kompositmaterialien in solchen Fällen erleichtert werden, bei welchen sich die nanopartikulären Calciumsalze schwer dispergieren lassen. Das Oberflächenmodifikationsmittel wird an die Oberfläche der Nanopartikel adsorbiert und verändert sie dergestalt, daß die Dispergierbarkeit des Calciumsalzes zunimmt und die Agglomeration der Nanopartikel verhindert wird.

Darüber hinaus kann durch eine Oberflächenmodifikation die Struktur der Kompositmaterialien sowie die Beladung der Proteinkomponente mit dem nanopartikulären Calciumsalz beeinflußt werden. Auf diese Weise ist es bei der Anwendung der Kompositmaterialien in Remineralisationsprozessen möglich, Einfluß auf den Verlauf , und die Geschwindigkeit des Remineralisationsprozesses zu nehmen.

Unter Oberflächenmodifikationsmitteln sind Stoffe zu verstehen, welche an der Oberfläche der feinteiligen Partikel physikalisch anhaften, mit diesen jedoch nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann auch unter den Begriffen Tenside und Schutzkolloide bekannt. Geeignete Tenside oder polymere Schutzkolloide können der deutschen Patentanmeldung DE 198 58 662 A1 entnommen werden.

Die Herstellung der erfindungsgemäßen Kompositmaterialien, in welchen die Primärpartikel der Calciumsalze oberflächenmodifiziert sind, kann nach analogen Fällungsverfahren wie vorstehend beschrieben erfolgen, wobei jedoch die Fällung der nanopartikulären Calciumsalze oder der Kompositmaterialien in Gegenwart eines oder mehrerer Oberflächenmodifikationsmittel erfolgt.

Bevorzugt wird zunächst durch eine Fällungsreaktion zwischen wäßrigen Lösungen von Calciumsalzen und wäßrigen Lösungen von Phosphat- und / oder Fluoridsalzen in Gegenwart der Oberflächenmodifikationsmittel die oberflächenmodifizierten nanopartikulären Calciumsalze erzeugt. Diese können anschließend von Begleitprodukten des Reaktionsgemischs gereinigt werden, z.B. durch Einengen unter reduziertem Druck und anschließende Dialyse. Durch Abziehen des Lösungsmittels kann zusätzlich eine Dispersion des oberflächenmodifizierten Calciumsalzes mit einem Feststoffanteil nach Wunsch hergestellt werden. Anschließend wird durch Zugabe der Proteinkomponenten in reiner, gelöster oder kolloidaler Form, wobei wiederum die Reihenfolge der Zugabe unkritisch ist, und erforperlichenfalls Nachreaktion bei erhöhter Temperatur, bevorzugt im Bereich zwischen 50 und 100 °C und für eine Dauer von 1 bis 100 Minuten, das Kompositmaterial aus oberflächenbeschichtetem Calciumsalz und Proteinkomponenten gebildet.

Für die Herstellung der erfindungsgemäßen Dental-Klebefolie wird der Wirkstoff, also das feinteilige, Kompositmaterial aus dem schwerlöslichen Calciumsalz und einer Proteinkomponente der noch flüssigen Lösung des Trägermaterials in Wasser oder wäßrigem Alkohol zugesetzt. Dazu kann der Wirkstoff als wasser- und lösungsmittelfreies Pulver oder auch als wäßrige oder wäßrig-alkoholische Dispersion verwendet werden. Schließlich wird die dabei erhaltene Dispersion in dünner Schicht auf einer nichthaftenden Unterlage getrocknet. Die Zugabemenge richtet sich dabei danach, wieviel von dem Wirkstoff in der fertigen Dental-Klebefolie enthalten sein soll. In einer bevorzugten Ausführung der Erfindung ist der Wirkstoff in einer Menge von 0,1-10 Gew.-% in der gebrauchsfertigen Dental-Klebefolie enthalten.

Zusätzlich zu dem erfindungsgemäß enthaltenen remineralisierenden, feinteiligen, in Wasser schwerlöslichen Caciumsalz können weitere Wirkstoffe, die für die Gesundheit der Zähne oder des Zahnfleisches förderlich und mit dem Trägermaterial verträglich sind, enthalten sein. Solche weiteren Wirkstoffe sind z.B.
- Karieshemmende Fluorverbindungen, z.B. Natriumfluorid, Zinnfluorid oder Natriummonofluorphosphat,
- Antizahnsteinwirkstoffe, z.B. Organophosphonate wie 1-Hydroxyethan-1,1-diphosphonsäure, Phosphonopropan-1,2,3-tricarbonsäure (Na-Salze), 1-Azacycloheptan-2,2-diphosphonsäure (Na-Salz),
- Desensibilisierende Wirkstoffe wie z.B. Kaliumnitrat oder Nelkenöl (Eugenol),
- Wundheilende und entzündungshemmende Stoffe wie z.B. Allantoin, Harnstoff, Azulen, Kamillenwirkstoffe, Rhodanid,
- Deodorierende und antimikrobielle Stoffe wie z.B. Chlorhexidin, Hexetidin, Bromchlorophen.

Weitere Hilfsmittel zur Verbesserung der organoleptischen Eigenschaften können ebenfalls enthalten sein, z.B.
- Aromaöle wie' z.B. Pfefferminzöl, Krauseminzöl, Eukalyptusöl, Anisöl, Fenchelöl, Kümmelöl, Fruchtaroma und synthetische Aromaöle,
- Süßungsmittel wie z.B. Saccharin-Natrium, Acesulfam-K, Aspartame^{®}, Natrium-Cyclamat, Stevioside, Thaumatin, Sucrose, Lactose, Maltose, Fructose oder Glycyrrhicin,
- Farbstoffe und Pigmente.

Die folgenden Beispiele sollen den Erfindungsgegenstand näher erläutern:

### Beispiele

### 1. Herstellung von Proteinlösungen bzw. -dispersionen

### 1.1 Gelatine Typ A :

10 g Gelatine Typ A (Gelatine gewonnen durch saure Hydrolyse von Schweinehaut) wurden mit 100 ml Wasser versetzt und mittels Mikrowelle einmal aufgekocht.

### 1.2 Gelatine Typ A und Casein:

10 g Gelatine Typ A wurden mit 100 ml Wasser sowie 10 ml des Überstands einer bei 20°C gesättigten und anschließend mit 5000 rpm zentrifugierten Caseinlösung versetzt und anschließend mittels Mikrowelle einmal aufgekocht.

### 1.3 Hydrolysat von Gelatine Typ A:

10 g Gelatine Typ A wurden mit 100 ml Wasser sowie der alkalischen Protease Savinase (Hersteller: Novo Nordisk) in einer Einsatzkonzentration von 0,005 % Enzym-Trockensubstanz, bezogen auf die Trockensubstanz der Gelantine, versetzt. Nach 20 h Rühren bei 20°C wurde mittels Mikrowelle einmal aufgekocht.

### 1.4 Hydrolysat von Gelatine Typ A und Casein:

10 g Gelatine Typ A und 1 g Casein wurden mit 100 ml H₂O versetzt, über Nacht bei Raumtemperatur mit alkalischer Protease Savinase (Hersteller: Novo Nordisk) in einer Einsatzkonzentration von 0,005 % Enzym-Trockensubstanz, bezogen auf die Trockensubstanz der Proteinkomponenten, hydrolysiert, dann einmal in der Mikrowelle aufgekocht und anschließend filtriert.

### 1.5 Gelatine Typ B :

10 g Gelatine Typ B (Gelatine gewonnen durch alkalische Hydrolyse von Rinderhaut) wurden mit 100 ml Wasser versetzt und mittels Mikrowelle einmal aufgekocht.

### 1.6 Gelatine Typ B und Casein:

10 g Gelatine Typ B wurden mit 100 ml Wasser sowie 10 ml des Überstands einer bei 20°C gesättigten und anschließend mit 5000 rpm zentrifugierten Caseinlösung versetzt und anschließend mittels Mikrowelle einmal aufgekocht.

### 1.7 Hydrolysat von Gelatine Typ B:

10 g Gelatine Typ B wurden mit 100 ml Wasser sowie der alkalischen Protease Savinase (Hersteller: Novo Nordisk) in einer Einsatzkonzentration von 0,005 % Enzym-Trockensubstanz, bezogen auf die Trockensubstanz der Gelatine, versetzt. Nach 20 h Rühren bei 20 °C wurde mittels Mikrowelle einmal aufgekocht.

### 1.8 Hydrolysat von Gelatine Typ B und Casein:

10 g Gelatine Typ B und 1 g Casein wurden mit 100 ml H2O versetzt, über Nacht bei Raumtemperatur mit alkalischer Protease Savinase (Hersteller: Novo Nordisk) in einer Einsatzkonzentration von 0,005 % Enzym-Trockensubstanz bezogen auf die Trockensubstanz der Proteinkomponenten hydrolysiert, dann einmal in der Mikrowelle aufgekocht und anschließend filtriert.

### 2.Herstellung von Kompositmaterialien durch Fällungsreaktionen in Gegenwart der Proteinkomponenten

### 2.1 Kompositmaterial aus Hydroxylapatit und Gelatine Typ A:

2,21 g Calciumchlorid wurden in 137 ml vollentsalztem Wasser gelöst, auf 25 °C temperiert und mit 25 Gew.-%iger wäßriger Ammoniaklösung auf pH = 11 eingestellt. Unter starkem Rühren wurden anschließend 20 ml der im Wasserbad auf 30-40 C erwärmten, nach Beispiel 1.1 hergestellten Proteinlösung zugefügt. Im Anschluß wurde eine wäßrige Lösung von 1,58 g Diammoniumhydrogenphosphat in 26 ml vollentsalztes Wasser, welche auf 25 °C temperiert und mit Ammoniaklösung auf pH = 11 eingestellt worden war, langsam innerhalb von 1 h zugetropft. Dabei fand die Ausfällung des Kompositmaterials statt. Der pH-Wert lag zu Beginn der Zutropfzeit bei 10,4 und wurde durch Nachdosierung von Ammoniaklösung bei einem Wert von ca. 10 gehalten. Nach 20 h Reaktionszeit (25°C, unter Rühren) war der pH-Wert der wäßrigen Suspension auf 9,5 abgefallen. Das ausgefallene Kompositmaterial wurde bei 5000 rpm abzentrifugiert mit ca. 30 - 40°C warmem vollentsalztem Wasser gewaschen und gefriergetrocknet. Man erhielt 2,2 g Kompositmaterial, dessen Elementaranalyse einen Kohlenstoffgehalt von 2,3 % ergab; dies entspricht einem Gehalt an Proteinmaterial von 5,6 Gew.-%, bezogen auf die Gesamtmenge des Kompositmaterials.

### 2.2-2.8 Kompositmaterialien aus Hydroxylapatit und weiteren Proteinkomponenten:

In analoger Weise wie unter Beispiel 2.1 beschrieben erhielt man Kompositmaterialien aus Hydroxylapatit sowie den unter 1.2 bis 1.8 beschriebenen Proteinkomponenten.

### 3. Herstellung von Kompositmaterialien durch Einarbeitung von Dispersionen oberflächenmodifizierter Calciumsalze in Proteinkomponenten

### 3.1 Kompositmaterial aus Hydroxylapatit und Gelatine Bloom 300:

### Zunächst wurden getrennt die Lösungen A und B hergestellt.

Lösung A: 25,4 g Calciumnitrat-tetrahydrat und 8,50 g Diammoniumhydrogenphosphat wurden jeweils in 100 g entionisiertem Wasser gelöst. Beide Lösungen wurden unter Ausbildung eines weißen Niederschlags zusammengegeben. Nach Zugabe von 10 ml 37 Gew.-%iger HCl erhielt man eine klare Lösung.

Lösung B: 200 ml entionisiertes Wasser, 200 ml 25 Gew.%ige wäßrige Ammoniaklösung sowie 20 g Plantacare^{®} 1200 wurden zusammengegeben und auf 0°C im Eisbad abgekühlt.

Unter Ausbildung eines Hydroxylapatit-Niederschlages gab man unter starkem Rühren Lösung A zu Lösung B. Nach Abziehen überschüssigen Ammoniaks wurde die Dispersion mittels Dialyse gereinigt.' Am Rotationsverdampfer engte man die Dispersion durch Bestimmung der abgeschiedenen Wassermenge anschließend soweit ein, daß der Feststoffanteil in der Dispersion, berechnet als Hydroxylapatit, 7.5 Gew.-% betrug.

Diese Dispersion wurde bei Raumtemperatur zu 100 ml g einer analog Beispiel 1.1 hergestellten 10 Gew.-%igen wässrigen Lösung von Gelatine Bloom 300 (Hersteller: Fluka) zugegeben, dann auf 80°C erwärmt und bei dieser Temperatur 5 Minuten gerührt. Anschließend ließ man die Masse unter Ausbildung des Kompositmaterials bei Raumtemperatur erstarren.

### 4. Herstellung von Dental-Klebefolien

### 4.1 PVAc/HPC-Folie

Es wurde eine Dispersion des Kompositmaterials in wäßrig alkoholischer Lösung von Polyvinylacetat und Hydroxypropylcellulose der folgenden Zusammensetzung hergestellt.

| | |
|---|---|
| Polyvinylacetat (MG 172000) | 5 Gew.-% |
| Hydroxypropylcellulose | 5 Gew.-% |
| Wasser | 9 Gew.-% |
| Methanol | 80 Gew.-% |
| Kompositmaterial | 1 Gew.-% |

Die Dispersion wurde in einer 2 m dicken Schicht auf eine silikonbeschichtete Unterlage gegossen und getrocknet. Es wurde ein ca. 0,2 mm dicker Film erhalten, der in 1 cm breite Bänder geschnitten wurde.

### 4.2 Gelatine-Folie

| | |
|---|---|
| Gelatine-Hydrolysat | 10,0 Gew.-% |
| Kompositmaterial gemäß Beispiel 3.1 | 1,0 Gew.-% |
| Ethanol | 45,0 Gew.-% |
| Wasser | 35,0 Gew.-% |
| Galloylgallussäure | 9,0 Gew.-% |

Die Dispersion wurde in 2 mm dicker Schicht auf eine silikonbeschichtete Unterlage gegossen und getrocknet. Es wurde ein ca. 0,2 mm dicker Film erhalten, der in ca. 1 cm breite Bänder geschnitten wurde.

## Patentansprüche

1. Dental-Klebefolie zur lokalen, remineralisierenden Zahnbehandlung, bestehend aus einem in Wasser löslichen oder quellbaren am Zahn haftenden Trägermaterial und darin eingelagerten Wirkstoffen, **dadurch gekennzeichnet, daß** als Wirkstoff ein feinteiliges, in Wasser schwerlösliches Calciumsalz, ausgewählt aus Fluoriden, Fluorphosphaten Hydroxylapatit, Fluorapatit und Gemischen davon, das gegebenenfalls auch Hydroxyl-, Carbonat- oder Chloridionen umfaßt, sowie eine Proteinkomponente, ausgewählt aus Gelatine, Casein, deren Hydrolysaten und Gemischen davon, enthalten ist, wobei der Wirkstoff als Kompositmaterial vorliegt, in welchem die Primärpartikel der Calciumsalze an das Gerüst der Proteinkomponente assoziiert sind.

2. Dental-Klebefolie gemäß Anspruch 1, **dadurch gekennzeichnet, daß** das feinteilige Calciumsalz eine mittlere Teilchenfeinheit von 10 bis 300 nm (Nanometer) aufweist.

3. Dental-Klebefolie gemäß Anspruch 1 oder 2, **dadurch gekennzeichnet, daß** der Wirkstoff in einer Menge von 0,1 bis 10 Gew.-%, bezogen auf die gebrauchsfertige Dental-Klebefolie, enthalten ist.

4. Dental-Klebefolie gemäß einem der Ansprüche 1 bis 3, **dadurch gekennzeichnet, daß** der Anteil der Proteinkomponenten im Kompositmaterial zwischen 0,1 und 60 Gew.-%, bevorzugt zwischen 1,0 und 20 Gew.-%, bezogen auf das Gewicht des Kompositmaterials, liegt.

5. Dental-Klebefolie gemäß einem der Ansprüche 1 bis 4, **dadurch gekennzeichnet, daß** als Trägermaterial ein wasserlösliches oder wasserquellbares, natürliches oder synthetisches Polymermaterial, ausgewählt aus pflanzlichen und mikrobiellen Gummen, Cellusoseethern, Copolymeren aus Acryl- oder Methacrylsäure und Estern der Acryl- oder Methacrylsäure, teilverseiftem Polyvinylacetat, Polyvinylpyrrolidon und Gemischen davon, Verwendung findet.

## Claims

1. Dental adhesive film for local, remineralizing tooth treatment, consisting of a carrier material adhering to the tooth and soluble or swellable in water, and active substances incorporated therein, **characterized in that** the active substance contains a finely divided calcium salt which is poorly soluble in water, selected from fluorides, fluorophosphates, hydroxyapatite, fluoroapatite and mixtures thereof, which optionally also comprises hydroxyl, carbonate or chloride ions, and a protein component selected from gelatin, casein, their hydrolysates and mixtures thereof, the active substance being present as a composite material in which the primary particles of the calcium salts are associated with the structure of the protein component.

2. Dental adhesive film according to Claim 1, **characterized in that** the finely divided calcium salt has a mean particle fineness of 10 to 300 nm (nanometres).

3. Dental adhesive film according to Claim 1 or 2, **characterized in that** the active substance is present in an amount of 0.1 to 10% by weight, based on the ready-to-use dental adhesive film.

4. Dental adhesive film according to one of Claims 1 to 3, **characterized in that** the proportion of the protein components in the composite material is between 0.1 and 60% by weight, preferably between 1.0 and 20% by weight, based on the weight of the composite material.

5. Dental adhesive film according to one of Claims 1 to 4, **characterized in that** the carrier material used is a water-soluble or water-swellable, natural or synthetic polymer material, selected from plant and microbial gums, cellulose ethers, copolymers of acrylic or methacrylic acid and esters of acrylic or methacrylic acid, partially hydrolysed polyvinyl acetate, polyvinylpyrrolidone and mixtures thereof.

## Revendications

1. Feuille adhésive dentaire pour le traitement dentaire local reminéralisant, constituée par un matériau support soluble ou apte au gonflement dans l'eau, adhérent à la dent et des substances actives qui y sont incorporées, **caractérisée en ce qu'**elle contient, comme substance active, un sel de calcium finement divisé, peu soluble dans l'eau, choisi parmi les fluorures, les fluorophosphates, l'hydroxylapatite, la fluoroapatite et les mélanges de ceux-ci, comprenant le cas échéant également des ions hydroxyle, carbonate ou chlorure, ainsi qu'un composant protéinique, choisi parmi la gélatine, la caséine, leurs hydrolysats et les mélanges de ceux-ci, la substance active se trouvant sous forme de matériau composite, dans lequel les particules primaires des sels de calcium sont associées à la structure du composant protéinique.

2. Feuille adhésive dentaire selon la revendication 1, **caractérisée en ce que** le sel de calcium finement divisé présente une finesse moyenne de particule de 10 à 300 nm (nanomètres).

3. Feuille adhésive dentaire selon la revendication 1 ou 2, **caractérisée en ce que** la substance active est contenue en une quantité de 0,1 à 10% en poids, par rapport à la feuille adhésive dentaire prête à l'emploi.

4. Feuille adhésive dentaire selon l'une quelconque des revendications 1 à 3, **caractérisée en ce que** la proportion de composants protéiniques dans le matériau composite est située entre 0,1 et 60% en poids, de préférence entre 1,0 et 20% en poids, par rapport au poids du matériau composite.

5. Feuille adhésive dentaire selon l'une quelconque des revendications 1 à 4, **caractérisée en ce qu'**on utilise, comme matériau support, un matériau polymère soluble ou apte au gonflement dans l'eau, naturel ou synthétique, choisi parmi les gommes végétales et microbiennes, les éthers de cellulose, les copolymères de l'acide acrylique ou méthacrylique et les esters de l'acide acrylique ou méthacrylique, le poly(acétate de vinyle) partiellement saponifié, la polyvinylpyrrolidone et les mélanges de ceux-ci.
